# EUROPÄISCHE PATENTSCHRIFT

(11) **EP 1 495 035 B1**
(45) Veröffentlichungstag und Bekanntmachung des Hinweises auf die Patenterteilung: **30.06.2010**
(21) Anmeldenummer: 03712030.0
(22) Anmeldetag: 17.03.2003
(51) Int. Cl.: B01F 17/00, B01J 31/02, C07C 211/63, C07D 231/12, C07D 233/54, C07D 233/56, C07D 249/08, C07F 9/30, C07F 9/54

(54) **VERFAHREN ZUR HERSTELLUNG VON BIS(PERFLUORALKYL)PHOSPHINSÄUREN UND DEREN SALZE**
METHOD FOR THE PRODUCTION OF BIS(PERFLUOROALKYL)PHOSPHINIC ACIDS AND THE SALTS THEREOF
PROCEDE DE PRODUCTION D'ACIDES BIS(PERFLUORO-AKLYL)PHOSPHINIQUES ET DE LEURS SELS

(30) Priorität: 16.04.2002 DE 10216997
(43) Veröffentlichungstag der Anmeldung: 12.01.2005
(73) Patentinhaber: Merck Patent GmbH, 64293 Darmstadt (DE)
(72) Erfinder: WELZ-BIERMANN, Urs, 64646 Heppenheim (DE); IGNATYEV, Nikolai, 47058 Duisburg (DE); WEIDEN, Michael, 64285 Darmstadt (DE); HEIDER, Udo, Winchester S022 4HZ (GB); KUCHERYNA, Andriy, 47053 Duisburg (DE); WILLNER, Helge, 45481 Mühlheim/Ruhr (DE); SARTORI, Peter, 86919 Utting (DE)
(86) Internationale Anmeldenummer: PCT/EP2003/002740
(87) Internationale Veröffentlichungsnummer: WO 2003/087110

(56) Entgegenhaltungen:
- WO-A-03/002579
- YAGUPOL' SKII L M: "Electrochemical fluorination of trialkylphosphine oxides" JOURNAL OF GENERAL CHEMISTRY OF THE USSR, CONSULTANTS BUREAU, NEW YORK, NY, US, Bd. 54, Nr. 4, PART 1, April 1984 (1984-04), Seiten 692-695, XP002125736 ISSN: 0022-1279
- CHEMICAL ABSTRACTS, vol. 84, no. 15, 12. April 1976 (1976-04-12) Columbus, Ohio, US; abstract no. 105768, SEMENII, V. YA. ET AL: "Bis (perfluoroalkyl)phosphinic acids" XP002246199 in der Anmeldung erwähnt & SU 498 311 T (INSTITUTE OF ORGANIC CHEMISTRY, ACADEMY OF SCIENCES, UKRAINIAN S.S.R.,) 5. Januar 1976 (1976-01-05)
- MAHMOOD, TARIQ ET AL: "Comparative study of tris(trifluoromethyl) phosphine oxide, tris(nonafluorobutyl) phosphine oxide and fluorobis(nonafluorobutyl) phosphine oxide with ammonia and amines" INORGANIC CHEMISTRY (1988), 27(17), 2913-16 , 1988, XP001148249 in der Anmeldung erwähnt
- PAVLENKO N.V. ET AL.: "Esters of bis(perfluoroalkyl)phosphinic acids" JOURNAL OF GENERAL CHEMISTRY USSR., Bd. 59, Nr. 3, 20. August 1989 (1989-08-20), Seiten 474-476, XP002246156 CONSULTANTS BUREAU. NEW YORK., US
- KALIBABCHUK N.N. ET AL: 'Use of 19F NMR for the investigation of micelle formation in aqueous solutions of ammonium perfluoroalkylphosphonate' THEORETICAL AND EXPERIMENTAL CHEMISTRY Bd. 11, Nr. 6, 1975, Seiten 700 - 703, XP009085723 ISSN: 0040-5760
- KALIBABCHUK N.N. ET AL: 'The effect of charge of the counterion on micellar and thermodynamic characteristics of anion-active SAM' SOVIET PROGRESS IN CHEMISTRY Bd. 44, Nr. 1, 1978, Seiten 65 - 67, XP009085719 ISSN: 0038-5743

## Beschreibung

Die vorliegende Erfindung betrifft ein Verfahren zur Herstellung von Bis(perfluoralkyl)phosphinsäuren umfassend zumindest die Umsetzung wenigstens eines Difluortris(perfluoralkyl)phosphorans oder wenigstens eines Trifluorbis(perfluoralkyl)phosphorans mit Fluorwasserstoff in einem geeigneten Reaktionsmedium und das Erhitzen des so erhaltenen Reaktionsgemisches. Die Erfindung betrifft auch Salze der Bis(perfluoralkyl)phosphinsäuren sowie deren Verwendungen.

Bis(perfluoralkyl)phosphinsäuren sind seit langem bekannt und eignen sich zur Herstellung verschiedener Chemikalien, wie beispielsweise entsprechender Methylester, die starke Methylierungsreagenzien darstellen (N. V. Pavlenko et al., Journal of General Chemistry of the USSR, 59, Nr. 3 (1989), Seiten 474-476).

Die Bis(perfluoralkyl)phosphinsäuren und ihre entsprechenden Salze finden ferner Verwendung aufgrund ihrer oberflächenaktiven Wirkung (DE-OS 22 33 941; N.N. Kalibabchuk et al., Theoretical and Exprimental Chemistry, 11, Nr. 6 (1975), Seiten 700-703; N.N. Kalibabchuk et al., Soviet Progress in Chemistry., 44, Nr. 1 (1978), Seiten 65-67) sowie in Brennstoffzellen (T. Mahmood, Inorganic Chemistry, 25 (1986), Seiten 3128-3131).

Das Lithiumsalz der Bis(pentafluorethyl)phosphinsäure ist ein vielversprechender Kandidat für die Verwendung als Leitsalz in Lithiumbatterien (F. Kita et al., Proc. Electrochem. Soc., 99-25, (2000), Seiten 480-484; F. Kita et al., J. Power Sources, 90, Nr. 1 (2000), Seiten 27-32).

Die Herstellung von Bis(trifluormethyl)phosphinsäure gelingt durch die Hydrolyse von schwer zugänglichem Bis(trifluormethyl)phosphortrichlorid

(H. J. Emeleus et al., J. Chem. Soc. (1955), Seiten 563-574). Die höheren Homologen der Bis(trifluormethyl)phosphinsäure wurden aus den entsprechenden Difluortris(perfluoralkyl)phosphoranen gewonnen (V. Ya. Semenii et al., U.S.S.R.-Patent 498,311).

In der Literatur sind im wesentlichen zwei verschiedene Verfahren zur Herstellung von Bis(perfluoralkyl)phosphinsäuren bekannt.

Gemäß dem ersten Verfahren erfolgt in einem ersten Schritt die Umsetzung eines Difluortris(perfluoralkyl)phosphorans mit Hexamethyldisiloxan zu dem entsprechenden Phosphinoxid. In einem zweiten Schritt folgt dann die Hydrolyse zu der entsprechenden Bis(perfluoralkyl)phosphinsäure. Dieses Verfahren weist den Nachteil auf, daß die Temperatur während der Hydrolyse sehr exakt kontrolliert und geregelt werden muß und üblicherweise nur geringste Mengen der gewünschten Bis(perfluoralkyl)phosphinsäure erhalten werden (T. Mahmood, Inorganic Chemistry, 25 (1986), Seiten 3128-3131; U.S.S.R.-Patent, 498,311; Seiten 57-61; T. Mahmood et al., Inorganic Chemistry, 27 (1988), Seiten 2913-2916).

Als weiteres Verfahren ist die direkte Hydrolyse von Difluortris(perfluoralkyl)phosphoranen zu Bis(perfluoralkyl)phosphinsäuren bekannt (T. Mahmood et al, Inorganic Chemistry, 27 (1988), Seiten 2913-2916). Nachteilig ist bei diesem Verfahren, daß die Hydrolyse aufgrund der sehr schlechten Mischbarkeit der Phosphorane mit Wasser, insbesondere der Phosphorane mit langen Alkylketten, nur sehr langsam abläuft und üblicherweise zu sehr komplexen Produktgemischen führt.

Die Aufgabe der vorliegenden Erfindung bestand daher darin, ein Verfahren zur Verfügung zu stellen, welches die einfache und kostengünstige Herstellung von Bis(perfluoralkyl)phosphinsäuren in guten Ausbeuten ermöglicht. Vorzugsweise sollen die Bis(perfluoralkyl)phosphinsäuren in hoher Reinheit erhalten werden. Eine weitere Aufgabe bestand in der Bereitstellung von Salzen der Bis(perfluoralkyl)phosphinsäuren.

Diese Aufgabe wurde durch das erfindungsgemäße Verfahren zur Herstellung von Bis(perfluoralkyl)phosphinsäuren gelöst, welches zumindest die folgenden Verfahrensschritte umfaßt:
a) Umsetzung wenigstens eines Difluortris(perfluoralkyl)phosphorans oder wenigstens eines Trifluorbis(perfluoralkyl)phosphorans mit Fluorwasserstoff in einem geeigneten Reaktionsmedium, und
b) Erhitzen des gemäß a) erhaltenen Reaktionsgemisches.

Die Herstellung der Difluortris(perfluoralkyl)phosphorane und Trifluorbis(perfluoralkyl)phosphorane kann nach üblichen, dem Fachmann bekannten Methoden erfolgen.
Vorzugsweise werden diese Verbindungen durch elektrochemische Fluorierung geeigneter Ausgangsverbindungen, hergestellt, wie in V. Ya. Semenii et al., Journal of General Chemistry of the USSR, 55, Nr. 12 (1985), Seiten 2415-2417; N. Ignatiev, J. of Fluorine Chem., 103 (2000), Seiten 57-61 sowie der WO 00/21969 beschrieben. Die entsprechenden Beschreibungen werden hiermit als Referenz eingeführt und gelten als Teil der Offenbarung.

Es können auch Mischungen aus zwei oder mehr Difluortris(perfluoralkyl)phosphoranen und/oder zwei oder mehr Trifluorbis(perfluoralkyl)phosphoranen in dem erfindungsgemäßen Verfahren zum Einsatz kommen. Vorzugsweise kommt jeweils nur ein Difluortris(perfluoralkyl)phosphoran oder ein Trifluorbis(perfluoralkyl)phosphoran in dem erfindungsgemäßen Verfahren zum Einsatz.

In einer bevorzugten Ausführungsform des erfindungsgemäßen Verfahrens kommt wenigstens ein Difluortris(perfluoralkyl)phosphoran oder wenigstens ein Trifluorbis(perfluoralkyl)phosphoran der allgemeinen Formel I 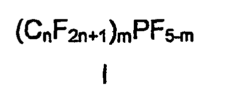 mit 1 ≤n ≤8, vorzugsweise 1 ≤n ≤4 und m jeweils =2 oder 3 zum Einsatz.

Besonders bevorzugte Difluortris(perfluoralkyl)phosphoranverbindungen können ausgewählt werden aus der Gruppe bestehend aus Difluortris(pentafluorethyl)phosphoran, Difluortris(n-nonafluorbutyl)phosphoran und Difluortris(n-heptafluorpropyl)phosphoran.

Als besonders bevorzugte Trifluorbis(perfluoralkyl)phosphoranverbindung kann Trifluorbis(n-nonafluorbutyl)phosphoran in dem erfindungsgemäßen Verfahren zum Einsatz kommen.
Die Umsetzung wenigstens eines Difluortris(perfluoralkyl)phosphorans oder wenigstens eines Trifluorbis(perfluoralkyl)phosphorans mit Fluorwasserstoff in einem geeigneten Reaktionsmedium erfolgt bevorzugt gemäß einem Verfahren, wie es in der WO 03/002579 beschrieben ist. Die entsprechende Beschreibung wird hiermit als Referenz eingeführt und gilt als Teil der Offenbarung.

Die Temperatur für das Erhitzen des gemäß Verfahrensschritt a) erhaltenen Reaktionsgemisches im Verfahrensschritt b) beträgt bevorzugt Raumtemperatur bis 150 °C, besonders bevorzugt 100 °C bis 145 °C und ganz besonders bevorzugt 135 bis 140 °C.

Vorzugsweise wird das nach Verfahrensschritt a) erhaltene Reaktionsgemisch gemäß Verfahrensschritt b) für 1 bis 150 Stunden, besonders bevorzugt für 10 bis 25 Stunden und ganz besonders bevorzugt für 18 bis 22 Stunden erhitzt.

Gegebenenfalls kann es vorteilhaft sein während des Erhitzens gemäß Verfahrensschritt b) erneut etwas von dem gleichen oder einem anderen Reaktionsmedium zu der Reaktionsmischung zu geben.

Um die Hydrolyse zu beschleunigen, kann man das gemäß Verfahrensschritt a) erhaltene Reaktionsgemisch bevorzugt auch in einer geschlossenen, druckdichten Vorrichtung, wie z.B. einem Autoklaven bei erhöhter Temperatur, vorzugsweise von 140 °C bis 200 °C erhitzen.

Neben den gewünschten Bis(perfluoralkyl)phosphinsäuren werden nach der Umsetzung gemäß dem erfindungsgemäßen Verfahren als weitere Reaktionsprodukte Fluorwasserstoff und jeweils das entsprechende Monohydroperfluoralkan erhalten.
Diese Reaktionsprodukte können nach üblichen, dem Fachmann geläufigen Verfahren ggf. abgetrennt, ggf. aufgefangen und ggf. isoliert werden, beispielsweise durch Kondensation in geeigneten Kühlfallen.

Fluorwasserstoff und Monohydroperfluoralkane stellen selbst wertvolle chemische Rohstoffe dar, die einer sinnvollen Nutzung zugeführt werden können. So ist es u.a. möglich, den Fluorwasserstoff aufzufangen und zu rezyklieren, so daß dieser für die Umsetzung gemäß Verfahrensschritt a) zur Verfügung steht.
Sofern erforderlich, kann sich an die Herstellung von Bis(perfluoralkyl)-phosphinsäuren nach dem erfindungsgemäßen Verfahren eine Reinigung und ggf. Isolierung dieser Verbindungen nach üblichen, dem Fachmann geläufigen Verfahren anschließen.

Vorzugsweise erfolgt die Reinigung durch Destillation, vorzugsweise unter vermindertem Druck bei erhöhten Temperaturen.

Die Isolierung der jeweiligen Salze der Bis(perfluoralkyl)phosphinsäure kann bevorzugt durch die Neutralisation der Bis(perfluoralkyl)phosphinsäuren erfolgen.

Die Darstellung der Salze aus der jeweiligen Bis(perfluoralkyl)phosphinsäure erfolgt durch Umsetzung mit wenigstens einer üblichen, dem Fachmann bekannten Base, vorzugsweise in Lösung.

Zur Darstellung der Salze werden die Bis(perfluoralkyl)phosphinsäuren mit Basen, vorzugsweise ausgewählt aus der Gruppe der Hydroxide, Oxide, Hydride, Amide, Carbonate, Phosphine oder Amine, neutralisiert.

Nach der Neutralisation wird das anfallende Salz, in dem Fachmann bekannter Weise, aufbereitet. Das Salz kann gewaschen und anschließend getrocknet werden.

Gegenstand der Anmeldung sind auch Salze der Bis(perfluoralkyl)phosphinsäuren, ausgewählt aus der Gruppe der teil- und peralkylierten Ammonium-, Phosphonium-, Sulfonium-, Pyridinium-, Pyridazinium-, Pyrimidinium-, Pyrazinium-, Imidazolium-, Pyrazolium-, Thiazolium-, Oxazolium- und Triazollumsalze-salze außer Triethylmethylammoniumbis(heptafluorpropyl)phosphinat und außer N-Methyl-2-trifluormethylbenzthiazoliumbis(heptafluorpropyl) phosphinat, die aus JOURNAL OF GENERAL CHEMISTRY OF THE USSR, 1989, 59(3), 474-476 bekannt sind. Bevorzugt werden Salze der Bis(perfluoralkyl)phosphinsäuren mit einem Kation ausgewählt aus der Gruppe 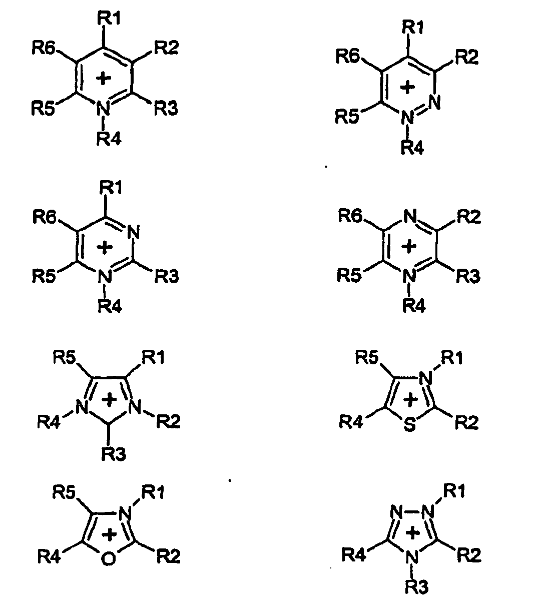 wobei R¹ bis R⁶ gleich oder verschieden, gegebenenfalls durch eine Einfach- oder Doppelbindung direkt miteinander verbunden sind und jeweils einzeln oder gemeinsam folgende Bedeutung haben:
- H,
- Halogen, wobei die Halogene nicht direkt mit N verbunden werden,
- Alkylrest (C₁ bis C₈) der teilweise oder vollständig durch weitere Gruppen, vorzugsweise F, Cl, N(CₙF₍₂ₙ₊₁₋ₓ)Hₓ₎₂, O(CₙF(₂ₙ₊₁₋ₓ₎Hₓ), SO₂(CₙF(₂ₙ₊₁₋ₓ)Hₓ), CₙF(₂ₙ₊₁₋ₓ)Hₓ mit 1<n<6 und 0<x≤2n+1 substituiert sein kann, dargestellt.

Überraschend wurde gefunden, daß diese Salze als ionische Flüssigkeiten, Phasen-Transfer-Katalysatoren oder Tenside verwendet werden können.

Das erfindungsgemäße Verfahren zur Herstellung von Bis(perfluoralkyl)-phosphinsäuren ermöglicht die einfache, kostengünstige und sichere Herstellung dieser Verbindungen in sehr guten Ausbeuten. Üblicherweise werden die Bis(perfluoralkyl)phosphinsäuren ohne weitere aufwendige Reinigungsschritte in hoher Reinheit erhalten. Durch die Umsetzung mit Basen können Salze gewonnen werden, die bisher nicht verfügbar waren

Vorteilhaft ist ferner, daß die gemäß dem erfindungsgemäßen Verfahren erhaltenen Nebenprodukte, nämlich Fluorwasserstoff und Monohydroperfluoralkane, selbst wertvolle Rohstoffe darstellen, die einer sinnvollen Nutzung zugeführt werden können. Hierdurch wird die Umweltbelastung bei der Umsetzung nach dem erfindungsgemäßen Verfahren gering gehalten und die Kosten für das erfindungsgemäße Verfahren reduziert.

Im folgenden wird die Erfindung anhand von Beispielen erläutert. Diese Beispiele dienen lediglich der Erläuterung der Erfindung und schränken den allgemeinen Erfindungsgedanken nicht ein.

### Beispiele

Die NMR-Spektren wurden mit einem Bruker Avance 300 NMR Spektrometer bei folgenden Frequenzen aufgenommen:
300,1 MHz ¹H
282,4 MHz für ¹⁹F und
121,5 MHz für ³¹P.

### Beispiel 1:

### Synthese von Bis(pentafluorethyl)phosphinsäure (C₂F₅)₂P(O)OH

### a)

Zu 2,93 g 40 % Gew.-iger Flusssäure (entsprechend 58,6 mmol HF) in einem FEP-(Fluorethylen-Polymer)-Kolben wurden 3,53 g Wasser gegeben (entsprechend einer Gesamtmenge an Wasser in der Mischung von 294 mmol). Die so erhaltene Mischung wurde dann mit einem Eisbad gekühlt. Anschließend wurden unter Rühren mit einem Magnetrührer 25,03 g (58,7 mmol) Difluortris(pentafluorethyl)phosphoran, (C₂F₅)₃PF₂, innerhalb von 3 Minuten zugegeben. Innerhalb von weiteren drei Minuten ging das Difluortris(pentafluorethyl)phosphoran vollständig in Lösung und es wurde eine farblose, klare Lösung von H⁺[(C₂F₅)₃PF₃]⁻ in Wasser erhalten.

Die so erhaltene Lösung wurde weitere 15 Minuten bei Raumtemperatur gerührt und anschließend bei einer Ölbadtemperatur von 135 bis 140 °C für 14 Stunden am Rückfluß erhitzt. Anschließend wurden weitere 4,83 g Wasser zu der Lösung gegeben und weitere 6 Stunden bei derselben Temperatur am Rückfluß erhitzt. Nach dem Abkühlen auf Raumtemperatur wurden 24,81 g einer klaren Lösung erhalten.

In einer zwischengeschalteten Falle, die mit Trockeneis gekühlt wurde, wurden 3,95 g einer zweiphasigen Flüssigkeit aufgefangen. Diese Flüssigkeit enthielt 2,11 g C₂F₅H, 1,5 g HF und 0,34 g der Ausgangsverbindung Difluortris(penta-fluorethyl)phosphoran.

Zur Isolierung der Bis(pentafluorethyl)phosphinsäure wurde wäßrige Fluorwasserstoff-Lösung aus dem Reaktionsgemisch abdestilliert, dabei wurden 15,13 g von nahezu reiner Bis(pentafluorethyl)phosphinsäure gewonnen. Die Ausbeute betrug 86,5 %, bezogen auf das eingesetzte Difluortris(pentafluorethyl)phosphoran.

Zur weiteren Reinigung wurde die Bis(pentafluorethyl)phosphinsäure unter vermindertem Druck bei 125 Pa destilliert. Der Siedepunkt betrug 63-64 °C.

Die so erhaltene Bis(pentafluorethyl)phosphinsäure wurde mittels ¹⁹F-, ³¹P- und ¹H-NMR-Spektroskopie sowie durch Elementaranalyse charakterisiert:
¹⁹F-NMR-Spektrum; δ, ppm:
   (Lösungsmittel Aceton-D₆, Referenz CCI₃F)
   -80,55 s (CF₃); -125,37 d (CF₂); J²_{P,F} = 78,2 Hz
¹H-NMR-Spektrum; δ, ppm:
   (Lösungsmittel Aceton-D₆ Referenz TMS)
   12,71 br. s (OH)
³¹P-NMR-Spektrum; δ, ppm:
   (Lösungsmittel Aceton-D₆, Referenz 85 Gew.-%ige H₃PO₄)
   -0,03 quin; J²_{P,F} = 78,3 Hz

Die Werte der gefundenen chemischen Verschiebungen entsprechen den aus der Veröffentlichung von T. Mahmood, Inorganic Chemistry, 25 (1986), Seiten 3128-3131 bekannten Werten.

Elementaranalyse:
Gefunden: C 15,76 %; H 0,40 %
Berechnet für ((C₂F₅)₂P(O)OH): C 15,91 %; H 0,33 %

### b)

Zu 0,834 g einer 40-Gew.%-igen, wässrigen Fluorwasserstoff-Säure (entsprechend 16,7 mmol HF) in einem FEP-Kolben wurden 2,50 g Wasser gegeben (entsprechend einer Gesamtmenge an Wasser in der Mischung von 166,5 mmol). Die so erhaltene Mischung wurde dann mit einem Eisbad gekühlt. Abschließend wurden unter Rühren mit einem Magnetrührer 7,11 g (16,7 mmol) Difluortris(pentafluorethyl)phosphoran, (C₂F₅)₃PF₂, innerhalb von drei Minuten zugegeben. Innerhalb von weiteren drei Minuten ging das Difluortris(pentafluorethyl)phosphoran vollständig in Lösung und es wurde eine farblose, klare Lösung von H⁺[(C₂F₅)₃PF₃]⁻ in Wasser erhalten. Die Reaktionsmischung wurde bei einer Ölbadtemperatur von 115 °C - 120 °C für 108 Stunden am Rückfluß erhitzt. Zur Isolierung der Bis(pentafluorethyl)phosphinsäure wurden Wasser-HF Lösung aus der Reaktionsmischung abdestilliert, wobei 3,97 g von nahezu reiner Bis(pentafluorethyl)phosphinsäure, (C₂F₅)₂P(O)OH, gewonnen wurden. Die Ausbeute betrug 78,8 %, bezogen auf das eingesetzte Difluortris(pentafluorethyl)phosphoran. Das so erhaltene Produkt wurde mittels ¹⁹F-NMR-Spektroskopie charakterisiert. Die entsprechenden Signale stimmten mit den unter Beispiel 1a genannten Signalen überein.

### c)

2,59 g (56,2 mmol) Ethanol wurden in einem FEP-Gefäß mit einem Eisbad gekühlt. Unter Rühren mit einem Magnetrührer wurden zuerst 0,49 g (24,5 mmol) Fluorwasserstoff (HF) langsam zu dem Ethanol gegeben und innerhalb von drei weiteren Minuten 9,59 g (22,5 mmol) Difluortris(pentafluorethyl)phosphoran, (C₂F₅)₃PF₂, zur Reaktionsmischung hinzugefügt. Nach dem Auflösen des Phosphorans wurden 2,21 g (122,6 mmol) Wasser zu der Lösung gegeben und Reaktionsmischung wurden bei einer Ölbadtemperatur von 120 °C für 144 Stunden am Rückfluß erhitzt (nach dem 44 Stunden wurden 2,1 g Wasser und nach 94 Stunden noch einmal 2,0 g Wasser zu der Reaktionsmischung gegeben).

Zur Isolierung der Bis(pentafluorethyl)phosphinsäure wurden Ethanol-Wasser-HF Lösing aus der Reaktionsmischung abbdestilliert, wobei 5.21 g von nahezu reiner Bis(pentafluorethyl)phosphinsäure, (C₂F₅)₂P(O)OH, gewonnen wurden. Die Ausbeute betrug 76,6 %, bezogen auf das eingesetzte Difluortris(pentafluorethyl)phosphoran. Das so erhaltene Produkt wurde mittels ¹⁹F-NMR-Spektroskopie charakterisiert. Die entsprechenden Signale stimmten mit den unter Beispiel 1 a angegebenen Signalen überein.

### Beispiel 2:

### Synthese von Bis(n-nonafluorbutyl)phosphinsäure (n-C₄F₉)₂P(O)OH

### a)

Zu 4,07 g einer 40-Gew.-%-igen Flusssäure (entsprechend 81,4 mol HF) in einem FEP-(Fluorethylen-Polymer)-Kolben wurden 4,25 g Wasser gegeben (entsprechend einer Gesamtmenge an Wasser in der Mischung von 371 mmol). Die so erhaltene Mischung wurde dann mit einem Eisbad gekühlt. Anschließend wurden unter Rühren mit einem Magnetrührer 51,42

g (70,8 mmol) Difluortris(n-nonafluorbutyl)phosphoran, (n-C₄F₉)₃PF₂, innerhalb von 10 Minuten zugegeben.

Die so erhaltene Lösung wurde weitere 20 Minuten bei Raumtemperatur gerührt und anschließend bei einer Ölbadtemperatur von 135 bis 140 °C für 11,5 Stunden am Rückfluß erhitzt. Anschließend wurden weitere 5,00 g Wasser zu der Lösung gegeben und weitere 8,5 Stunden bei derselben Temperatur am Rückfluß erhitzt. Nach dem Abkühlen auf Raumtemperatur wurden 46,47 g einer klaren Lösung erhalten.
In einer zwischengeschalteten Falle, die mit Trockeneis gekühlt wurde, wurden 15,03 g einer zweiphasigen Flüssigkeit aufgefangen. Diese Flüssigkeit enthielt 13,06 g n-C₄F₉H und 1,97 g HF (obere Phase).

Zur Isolierung der Bis(n-nonafluorbutyl)phosphinsäure wurde wäßrige Fluorwasserstoff-Lösung aus dem Reaktionsgemisch bei einer Ölbadtemperatur von 145 °C abdestilliert, und 34,62 g nahezu reiner Bis(n-nonafluorbutyl)phosphinsäure als Feststoff gewonnen. Die Ausbeute betrug 97,4 %, bezogen auf das eingesetzte Difluortris(n-nonafluorbutyl)phosphoran.

Zur weiteren Reinigung wurde die Bis(n-nonafluorbutyl)phosphinsäure unter vermindertem Druck bei 125 Pa destilliert. Der Siedepunkt betrug 124 °C. Beim Abkühlen erstarrt die so erhaltene Bis(n-nonafluorbutyl)phosphinsäure zu einem Feststoff mit einem Schmelzpunkt von 103-104 °C.

In der Literaturveröffentlichung von T. Mahmood, Inorganic Chemistry, 25 (1986), Seiten 3128-3131 wird die Bis(n-nonafluorbutyl)phosphinsäure als nichtflüchtige Flüssigkeit beschrieben, wobei es sich wahrscheinlich um eine hydratisierte Form dieser Verbindung handelt.

Die Bis(n-nonafluorbutyl)phosphinsäure wurde mittels ¹⁹F-, ³¹P- und ¹H-NMR-Spektroskopie sowie durch Elementaranalyse charakterisiert:
¹⁹F-NMR-Spektrum; δ, ppm:
   (Lösungsmittel Aceton-D₆, Referenz CCl₃F)
   -80,90 t (CF₃); -120,50 br. s (CF₂); -121,38 d (CF₂); -125,58 m (CF₂); J²_{P,F} = 79,5 Hz, J⁴_{F,F} = 9,9 Hz
¹H-NMR-Spektrum; δ, ppm:
   (Lösungsmittel Aceton-D₆, Referenz TMS)
   9,25 br. s (OH)
³¹P-NMR-Spektrum; δ, ppm:
   (Lösungsmittel Aceton-D₆, Referenz 85 Gew.-%ige H₃PO₄)
   1,74 quin; J²_{P,F} = 79,0 Hz

Die Werte der gefundenen chemischen Verschiebungen entsprechen den aus der Veröffentlichung von T. Mahmood, Inorganic Chemistry, 25 (1986), Seiten 3128-3131 bekannten Werten.

Elementaranalyse:
Gefunden: C 19,05 %; H 0,20 %
Berechnet für ((n-C₄F₉)₂P(O)OH): C 19,14 %; H 0,20 %

### b)

Zu 1,08 g einer 40-Gew.%-igen, wässrigen Fluorwasserstoff-Säure (entsprechend 21,6 mmol HF) in einem FEP-Kolben wurden 1,45 g Wasser gegeben (entsprechend einer Gesamtmenge an Wasser in der Mischung von 116,1 mmol). Die so erhaltene Mischung wurde dann mit einem Eisbad gekühlt. Abschließend wurden unter Rühren mit einem Magnetrührer 7,98 g (15.2 mmol) Trifluorbis(n-nonafluorbutyl)phosphoran, (C₄F₉)₂PF₃, innerhalb von 10 Minuten zugegeben. Die Reaktionsmischung wurde 15 Stunden bei Raumtemperatur gerührt und anschließend bei einer Ölbadtemperatur von 110°C für 35 Stunden am Rückfluß erhitzt (nach 17 Stunden wurden weitere 0,6 g Wasser und nach 25 Stunden weitere 1,2 g Wasser zu der Reaktionsmischung gegeben). Zur Isolierung der Bis(n-nonafluorbutyl)phosphinsäure wurden Wasser-HF Lösung aus der Reaktionsmischung abdestilliert, wobei 6,34 g von nahezu reiner Bis(n-nonafluorobutyl)phosphinsäure gewonnen wurden. Die Ausbeute betrug 83,2 %, bezogen auf das eingesetzte Trifluorbis(n-nonafluorbutyl)phosphoran. Das so erhaltene Produkt wurde mittels ¹⁹F-NMR-Spektroskopie charakterisiert. Die entsprechenden Signale stimmten mit den unter Beispiel 2a genannten Signalen überein.

### Beispiel 3:

### 3,07 g der gemäß Beispiel 1 hergestellten

Bis(pentafluorethyl)phosphinsäure wurden in 50 cm³ Wasser mit 7,48 g einer 20 Gew.-%igen, wäßrigen Lösung von Tetraethylammoniumhydroxid neutralisiert. Anschließend wurde das Wasser abgedampft und der so erhaltene Rückstand unter vermindertem Druck von 120 Pa bei 70 °C (Badtemperatur) getrocknet.
Es wurden 4,38 g weißer Feststoff von Tetraethylammonium-bis(pentafluorethyl)phosphinat mit einem Schmelzpunkt von 100-102 °C erhalten. Die Ausbeute ist nahezu quantitativ, bezogen auf die eingesetzte Bis(pentafluorethyl)phosphinsäure.

Das Tetraethylammoniumbis(pentafluorethyl)phosphinat wurde mittels ¹⁹F-, ³¹P- und ¹H-NMR-Spektroskopie sowie durch Elementaranalyse charakterisiert:
¹⁹F-NMR-Spektrum; δ ppm:
   (Lösungsmittel Aceton-D₆, Referenz CCl₃F)
   -80,23 s (CF₃); -124,90 d (CF₂); J²_{P,F} = 64,8 Hz
¹H-NMR-Spektrum; δ, ppm:
   (Lösungsmittel Aceton-D₆, Referenz TMS)
   1,36 tm (CH₃); 3,48 q (CH₂); J³_{H,H} = 7,3 Hz
³¹P-NMR-Spektrum; δ, ppm:
   (Lösungsmittel Acoton-D₆, Referenz 85 Gew.-%ige H₃PO₄)
   0,28 quin, J²_{P,F} = 64,5 Hz
Elementaranalyse:
   Gefunden: C 33,36 %; H 4,60 %; N 3,22 %
   Berechnet für (C₂F₅)₂P(O)ON(C₂H₅)₄: C 33,42 %; H 4,67 %; N 3,25 %

### Beispiel 4:

### 2,52 g der gemäß Beispiel 1 hergestellten

Bis(pentafluorethyl)phosphinsäure wurden in 20 cm³ Wasser mit 0,577 g Kaliumcarbonat neutralisiert. Anschließend wurde das Wasser verdampft und der so erhaltene Rückstand unter vermindertem Druck bei 120 Pa und einer Badtemperatur von 100 °C getrocknet. Es wurden 2,83 g weißer Feststoff von Kaliumbis(pentafluorethyl)phosphinat erhalten. Die Ausbeute ist nahezu quantitativ, bezogen auf die eingesetzte Bis(pentafluorethyl)phosphinsäure. Das Salz zersetzte sich bei einer Temperatur von 203-205 °C.

Das Kallumbis(pentafluorethyl)phosphinat wurde mittels ¹⁹F- und ³¹P-NMR-Spektroskopie sowie durch Elementaranalyse charakterisiert:
¹⁹F-NMR-Spektrum; δ, ppm:
   (Lösungsmittel Aceton-D₆, Referenz CCl₃F)
   -80,40 m (CF₃); -125,11 d (CF₂); J²_{P,F}=67,4 Hz
³¹P-NMR-Spektrum; δ, ppm:
   (Lösungsmittel Aceton-D₆, Referenz 85 Gew.-%ige H₃PO₄)
   0,73 quin; J²_{P,F} = 67,2 Hz
Elementaranalyse:
   Gefunden: C 14,6 %;
   Berechnet für (C₂F₅)₂P(O)OK: C 14,13 %

### Beispiel 5:

4,00 g der gemäß Beispiel 2 hergestellten Bis(n-nonafluorbutyl)-phosphinsäure wurden in 50 cm³ Wasser mit 5,86 g einer 20 Gew.-%-igen wäßrigen Lösung von Tetraethylammoniumhydroxid neutralisiert. Hierbei bildete sich ein weißer Niederschlag, der abfiltriert und bei vermindertem Druck von 120 Pa und einer Badtemperatur von 70 °C getrocknet wurde. Es wurden 4,93 g weißer Feststoff von Tetraethylammoniumbis-(n-nonafluorbutyl)phosphinat mit einem Schmelzpunkt von 99-100 °C erhalten. Die Ausbeute betrug 98 %, bezogen auf die eingesetzte Bis(n-nonafluorethyl)phosphinsäure.

Das Tetraethylammoniumbis(n-nonafluorbutyl)phosphinat wurde mittels ¹⁹F-, ³¹P- und ¹H-NMR-Spektroskopie sowie durch Elementaranalyse charakterisiert:
¹⁹F-NMR-Spektrum; δ, ppm:
   (Lösungsmittel Aceton-D₆ Referenz CCl₃F)
   -80,75 tt (CF₃); -120,35 m (CF₂); -121,17 dm (CF₂); -125,30 m (CF₂); J²_{P,F} = 65,0 Hz; J⁴_{F,F} = 9,9 Hz, J_{F,F} = 3,1 Hz
¹H-NMR-Spektrum; δ, ppm:
   (Lösungsmittel Aceton-D₆, Referenz TMS)
   1,37 tm (CH₃), 3,48 q (CH₂); J³_{H,H} = 7,3 Hz
³¹P-NMR-Spektrum; δ, ppm:
   (Lösungsmittel Aceton-D₆, Referenz 85 Gew.-%ige H₃PO₄)
   1,70 quin; J²_{P,F} = 64,9 Hz
Elementaranalyse:
   Gefunden: C 30,32 %; H 3,05 %, N 2,10
   Berechnet für (n-C₄F₉)₂P(O)ON(C₂H₅)₄: C 30,44 %; H 3,19 %, N 2,22

### Beispiel 6:

1,93 g (6,39 mmol) der gemäß Beispiel 1 hergestellten Bis(pentafluorethyl)-phosphinsäure wurden in 50 cm³ Wasser mit einer Lösung von 0,371 g (3,19 mmol) 1,6-Diaminohexan in 15 cm³ Wasser neutralisiert. Das Wasser wurde abgedampft und der so erhaltene Rückstand unter vermindertem Druck bei 120 Pa und einer Badtemperatur von 100 °C getrocknet. Es wurden 2,21 g weißer Feststoff von Hexamethylen-1,6-Diammoniumbis(pentafluorethyl)phosphinat mit einem Schmelzpunkt von 208-210 °C erhalten. Die Ausbeute betrug 96,1 %, bezogen auf die eingesetzte Bis(pentafluorethyl)phosphinsäure.

Das Hexamethylen-1,6-Diammoniumbis(pentafluorethyl)phosphinat wurde mittels ¹⁹F-, ³¹P- und ¹H-NMR-Spektroskopie sowie durch Elementaranalyse charakterisiert:
¹⁹F-NMR-Spektrum; δ, ppm:
   (Lösungsmittel DMSO-D₆, Referenz CCl₃F)
   -79,59 m (CF₃); -124,66 ppm d (CF₂); J²_{P,F} = 65,6 Hz
¹H-NMR-Spektrum; δ ppm:
   (Lösungsmittel DMSO-D₆, Referenz TMS)
   1,30 m (2CH₂); 1,51 m (2CH₂); 2,76 m (2CH₂), 7,53 br. s (2NH₃⁺)
³¹P-NMR-Spektrum, δ, ppm:
   (Lösungsmittel OMSO-D₆, Referenzsubstanz 85 Gew.-%ige H₃PO₄)
   -2,15 quin; J²_{P,F} = 65,5 Hz
Elementaranalyse:
   Gefunden: C 23,61 %; H 2,49 %, N: 4,07 %
   Berechnet für [(C₂F₅)₂P(O)O]₂²⁻ [H₃N(CH₂)₆NH₃]²⁺ C 23,35 %; H 2,52 %; N 3,89 %

### Beispiel 7:

2,80 g (5,58 mmol) der gemäß Beispiel 2 hergestellten Bis(n-nonafluorbutyl)-phosphinsäure wurden in 50 cm³ Wasser mit einer Lösung von 0,324 g
(2,79 mmol) 1,6-Diaminohexan in 15 cm³ Wasser neutralisiert. Hierbei bildete sich ein weißer Niederschlag, der abfiltriert und unter vermindertem Druck bei 120 Pa und einer Badtemperatur von 100 °C getrocknet wurde. Es wurden 2,87 g weißer Feststoff von Hexamethylen 1,6-Diammoniumbis(n-nonafluorbutyl)-phosphinat mit einem Schmelzpunkt > 250 °C erhalten. Die Ausbeute betrug 92 %, bezogen auf die eingesetzte Bis(n-nonafluorbutyl)phosphinsäure.

Das Hexamethylen-1,6-Diammoniumbis(n-nonafluorbutyl)phosphinat wurde mittels ¹⁹F-, ³¹P- und ¹H-NMR-Spektroskopie sowie durch Elementaranalyse charakterisiert:
¹⁹F-NMR-Spektrum; δ, ppm:
   (Lösungsmittel DMSO-D₆, Referenz CCl₃F)
   -80,03 t (CF₃); -120,46 m (CF₂); -121,28 dm (CF₂), -125,11 m (CF₂), J²_{P,F} = 65,6 Hz, J⁴_{F,F} = 9,5 Hz
¹H-NMR-Spektrum; δ, ppm:
   (Lösungsmittel DMSO-D₆, Referenz TMS)
   1,29 m (2CH₂); 1,51 m (2CH₂); 2,76 m (2CH₂), 7,61 br. s (2NH₃⁺)
³¹P-NMR-Spektrum; δ, ppm:
   (Lösungsmittel DMSO-D₆, Referenz 85 Gew.-%ige H₃PO₄)
   -0,76 quin; J²_{P,F} = 65,5 Hz
Elementaranalyse:
   Gefunden: C 23,76 %; H 1,58 %; N 2,48 %
   Berechnet für: [(C₄F₉)₂P(O)O]₂²⁻ [H₃N(CH₂)₆NH₃]²⁺ C 25,59; H 1,62 %; N 2,50 %

### Beispiel 8:

2,23 g (4,44 mmol) der gemäß Beispiel 2 hergestellten Bis(n-nonafluorbutyl)-phosphinsäure wurden in 50 cm³ Wasser mit einer Lösung von 1,20 g (4,45 mmol) Tri-n-Hexylamin in 20 cm³ eines 1:1 (Vol/Vol) Wasser/Ethanol-Gemisches neutralisiert. Anschließend wurden 15 cm³ Ethanol zugegeben und 5 Minuten am Rückfluß gekocht.
Nach dem Abkühlen auf Raumtemperatur bildete sich ein weißer Niederschlag, der abfiltriert und unter vermindertem Druck bei 120 Pa und einer Badtemperatur von 60 °C getrocknet wurde. Es wurden 3,22 g weißer Feststoff von Tri-n-Hexylammoniumbis(n-nonafluorbutyl)phosphinat mit einem Schmelzpunkt von 74-75 °C erhalten. Die Ausbeute betrug 93,9 %, bezogen auf die eingesetzte Bis(n-nonafluorbutyl)phosphinsäure.
Das Tri-n-Hexylammoniumbis(n-nonafluorbutyl)phosphinat wurde mittels ¹⁹F, ³¹P- und ¹H-NMR-Spektroskopie sowie durch Elementaranalyse charakterisiert:
¹⁹F-NMR-Spektrum; δ, ppm:
   (Lösungsmittel Aceton-D₆, Referenz CCl₃F)
   -80,82 tt (CF₃); -120,36 m (CF₂); -121,32 dm (CF₂), -125,53 m (CF₂); J²_{P,F} = 70,1 Hz; J⁴_{F,F} = 9,9 Hz, J_{F,F} = 3,0 Hz
¹H-NMR-Spektrum; δ, ppm:
   (Lösungsmittel Aceton-D₆, Referenz TMS)
   0,89 m (3CH₂); 1,35 m (9CH₂); 1,82 m (3CH₂); 3,21 m (2CH₂); 9,62 br. s (NH⁺)
³¹P-NMR-Spektrum, δ, ppm:
   (Lösungsmittel Aceton-D₆, Referenz 85 Gew.-%ige H₃PO₄)
   1,76 quin; J²_{P,F} = 70,1 Hz
Elementaranalyse:
   Gefunden: C 40,51 %; H 5,20 %; N 1,80 %
   Berechnet für (C₄F₉)P(O)O^{- +}HN(C₆H₁₃)₃: C 40,45 %; H 5,22%; N 1,82 %

### Beispiel 9:

1,55 g (3,09 mmol) der gemäß Beispiel 2 hergestellten Bis(n-nonafluorbutyl)phosposinsäure werden in 15 cm³ Wasser mit einer Lösung von 1,20 g (3,09 mmol) Triphenyl-Benzylphosphoniumchlorid in 30 cm³ Wasser gemischt und 5 min bei Raumtemperatur gerührt. Hierbei bildete sich ein weißer Niederschlag, der abfiltriert und unter verminderten Druck bei 120 Pa und einer Badtemperatur von 60 °C getrocknet wird. Es werden 2,50 g weißer Feststoff von Triphenyl-Benzylphosphoniumbis(n-nonafluorbutyl)phosphinat mit einem Schmelzpunkt von 138-139 °C erhalten. Die Ausbeute beträgt 95,1 % auf die eingesetzte Bis(n-nonafluorbutyl)phosphinsäure,

Triphenylbenzylphosphoniumbis(n-nonafluorbutyl)phosphinat wird mittels ¹⁹F-, ³¹P- und ¹H-NMR-Spektroskopie sowie durch Elementaranalyse charakterisiert:
¹⁹F NMR spectrum, δ, ppm:
   (Lösungsmittel: Aceton-D₆ ; Referenz: CCl₃F):
      -80.76 t (CF₃); -120.36 m (CF₂); -121.21 dm (CF₂); -125.38 m (CF₂) ; J²_{P,F}=65.9 Hz, J⁴_{F,F}=9.9 Hz.
¹H NMR spectrum, δ, ppm:
   (Lösungsmittel: Aceton-D₆ ; Referenz: TMS):
      5.22 d (CH₂, PhCH₂) ; 7.11-7.17 m (2H, PhCH₂) ; 7.19-7.27 m (2H, PhCH₂)
      7.30-7.37 m (iH, PhCH₂) ; 7.72-7.87 m (12H, 3Ph) ; 7.91-7.99 m (3H, 3Ph) J²_{P,H}=15.1 Hz.
³¹P NMR spectrum, δ, ppm:
   (Lösungsmittel: Aceton-D₆ ; Referenz:85 Gew-%ige H₃PO₄):
      1.78 quin; 25.68 br.s ; J²_{P,F}=65.8Hz
Elementaranalyse:
   Gefunden: C 46.10%; H 2.48%.
   Berechnet für [(C₄F₉)₂P(O)O]⁻ [(C₆H₅)₃C₆H₅CH₂P]⁺: C 46.39 % ; H 2.60 %.

### Beispiel 10:

Zu 4,05 g (11,9 mmol) der gemäß Beispiel 4 hergestellten Kalium-bis(pentafluoroethyl)phosphinat in 15 cm³ Wasser wird unter ständigem Rühren eine Lösung von 2,08 g (11,9 mmol) 1-Butyl-3-methylimidazoliumchloride in 3 cm³ Wasser bei Raumtemperatur gegeben. Hierbei bildete sich ein öliger Niederschlag. Das Wasser wird unter vermindertem Druck abgedampft und der so erhaltene Rückstand unter vermindertem Druck von 120 Pa und einer Badtemperatur von 60 °C für 1 Stunde getrocknet. Anschließend werden 10 cm³ Isopropylalkohol zum Rückstand gegeben und ein weißer Niederschlag abfiltriert, der zwei Mal mit 5 cm³ Isopropylalkohol gewaschen wird. Der Isopropylalkohol wird unter vermindertem Druck abgedampft und der so erhaltene Rückstand unter vermindertem Druck von 120 Pa und einer Badtemperatur von 80 °C für 2 Stunden getrocknet.
Es werden 4.99 g einer öliger Flüssigkeit von 1-Butyl-3-methylimidazoliumbis(pentafluoroethyl)phosphinat gewonnen. Die Ausbeute beträgt 95,4 % , bezogen auf das eingesetzte Kaliumbis(pentafluoroethyl)-phosphinat.
1-Butyl-3-methylimidazoliumbis(pentafluoroethyl)phosphinat wurde mittels ¹⁹F, ³¹P und ¹H-NMR-Spektroskopie charakterisiert:
¹⁹_{F} NMR spectrum, ppm :
   (Lösungsmittel: Acetonitril-D₃ ; Referenz: CCl₃F):
      - 80,19 m (CF₃) ; -124,93 d (CF₂) ; J²_{P,F} = 66.9 Hz .
¹H NMR spectrum, ppm:
   (Lösungsmittel: Acetonitril-D₃ ; Referenz: TMS):
      0,93 t (3H, CH₃) ; 1,33 tq (2H, CH₂) ; 1,83 tt (2H, CH₂) ; 3,87 s (3H, CH₃) ; 4,17 t (2H, CH₂) ; 7.48 dd (1H) ; 7.54 dd (1H) ; 8.99 s (1H) ; J³_{H,H} = 1.6 Hz;
      J³_{H,H} = 7.3 Hz ; J³_{H,H} = 7.6 Hz.
³¹P NMR spectrum, ppm:
   (Lösungsmittel: Acetonitril-D₃ ; Referenz: 85 % H₃PO₄):
      - 1.86 quin ; .J²_{P,F} =66.8 Hz.

## Patentansprüche

1. Verfahren zur Herstellung von Bis(perfluoralkyl)phosphinsäuren bzw. deren Salzen umfassend zumindest die folgenden Verfahrensschritte:
a) Umsetzung wenigstens eines Difluortris(perfluoralkyl)phosphorans oder wenigstens eines Trifluorbis(perfluoralkyl)phosphorans mit Fluorwasserstoff in einem geeigneten Reaktionsmedium, und
b) Erhitzen des gemäß a) erhaltenen Reaktionsgemisches.

2. Verfahren zur Herstellung von Bis(perfluoralkyl)phosphinsäuren bzw. deren Salzen gemäß Anspruch 1, **dadurch gekennzeichnet, daß** die Salze durch anschließende Neutralisation dargestellt werden.

3. Verfahren gemäß Anspruch 1, **dadurch gekennzeichnet, daß** als Difluortris(perfluoralkyl)phosphoran oder Trifluorbis(perfluoralkyl)phosphoran eine Verbindung der allgemeinen Formel I 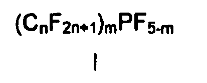 eingesetzt wird, worin 1 ≤ n ≤ 8, vorzugsweise 1 ≤ n ≤ 4 und m jeweils = 2 oder 3 bedeutet.

4. Verfahren gemäß Anspruch 1, **dadurch gekennzeichnet, daß** als Difluortris(perfluoralkyl)phosphoran eine Verbindung ausgewählt aus der Gruppe bestehend aus Difluortris(pentafluorethyl)phosphoran, Difluortris(n-nonafluorbutyl)phosphoran und Difluortris(n-heptafluorpropyl)phosphoran eingesetzt wird.

5. Verfahren gemäß Anspruch 1, **dadurch gekennzeichnet, daß** als Trifluorbis(perfluoralkyl)phosphoranverbindung Trifluorbis-(n-nonafluorbutyl)phosphoran eingesetzt wird.

6. Verfahren gemäß Anspruch 1, **dadurch gekennzeichnet, daß** die Temperatur während des Erhitzens gemäß Verfahrensschritt b) Raumtemperatur bis 150 °C, vorzugsweise 100 °C bis 145 °C, besonders bevorzugt 135 bis 140 °C, beträgt.

7. Verfahren gemäß Anspruch 1, **dadurch gekennzeichnet, daß** die Dauer des Erhitzens gemäß Verfahrensschritt b) 1 bis 150 Stunden, vorzugsweise 10 bis 25 Stunden, besonders bevorzugt 18 bis 22 Stunden beträgt.

8. Verfahren gemäß Anspruch 1, **dadurch gekennzeichnet, daß** das Reaktionsmedium Wasser oder ein auf Wasser basierendes Gemisch ist.

9. Verfahren gemäß Anspruch 2, **dadurch gekennzeichnet, daß** zur Darstellung der Salze Basen, vorzugsweise Hydroxide, Oxide, Hydride, Amide, Carbonate, Phosphine oder Amine, verwendet werden.

10. Salze der Bis(perfluoralkyl)phosphinsäuren ausgewählt aus der Gruppe der teil- und peralkylierten Ammonium-, Phosphonium-, Sulfonium-, Pyridinium-, Pyridazinium-, Pyrimidinium-, Pyrazinium-, Imidazolium-, Pyrazolium-, Thiazolium-, Oxazolium- und Triazoliumsalze-salze außer Triethylmethylammoniumbis-(heptafluorpropyl)phosphinat und außer N-methyl-2-trifluormethylbenzthiazoliumbis(heptafluorpropyl)phosphinat.

11. Salze der Bis(perfluoralkyl)phosphinsäuren gemäß Anspruch 10, mit einem Kation ausgewählt aus der Gruppe 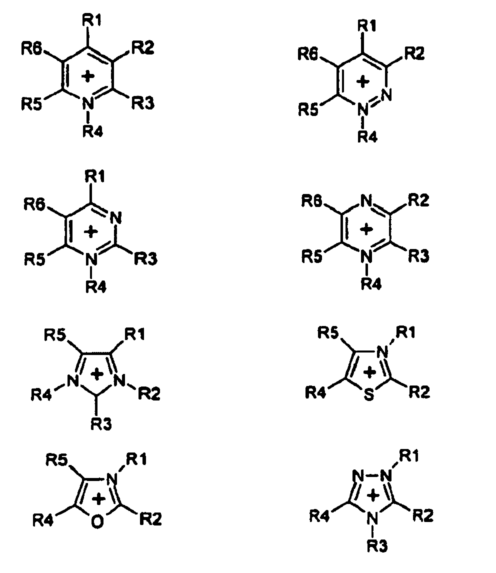 wobei R¹ bis R⁶ gleich oder verschieden, gegebenenfalls durch eine Einfach- oder Doppelbindung direkt miteinander verbunden sind und jeweils einzeln oder gemeinsam folgende Bedeutung haben:
- H,
- Halogen, wobei die Halogene nicht direkt mit N verbunden werden,
- Alkylrest (C₁ bis C₈) der teilweise oder vollständig durch weitere Gruppen, vorzugsweise F, Cl, N(CₙF₍₂ₙ₊₁₋ₓ)Hₓ)₂, O(CₙF₍₂ₙ₊₁₋ₓ)Hₓ), SO₂(CₙF₍₂ₙ₊₁₋ₓ₎Hₓ), CₙF₍₂ₙ₊₁₋ₓ₎Hₓ mit 1<n<6 und 0<x≤2n+ substituiert sein kann,
außer N-methyl-2-trifluormethylbenzthiazoliumbis(heptafluorpropyl)phosphinat.

12. Verwendung der Salze der Bis(perfluoralkyl)phosphinsäuren gemäß Anspruch 10 oder 11 als ionische Flüssigkeiten.

13. Verwendung der Salze der Bis(perfluoralkyl)phosphinsäuren gemäß Anspruch 10 oder 11 als Phasen-Transfer-Katalysator oder Tenside.

## Claims

1. Process for the preparation of bis(perfluoroalkyl)phosphinic acids or salts thereof comprising at least the following process steps:
a) reaction of at least one difluorotris(perfluoroalkyl)phosphorane or at least one trifluorobis(perfluoroalkyl)phosphorane with hydrogen fluoride in a suitable reaction medium, and
b) heating of the reaction mixture obtained in a).

2. Process for the preparation of bis(perfluoroalkyl)phosphinic acids or salts thereof according to Claim 1, **characterised in that** the salts are prepared by subsequent neutralisation.

3. Process according to Claim 1, **characterised in that** the difluorotris(perfluoroalkyl)phosphorane or trifluorobis(perfluoroalkyl)phosphorane employed is a compound of the general formula I 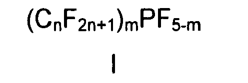 in which 1 ≤ n ≤ 8, preferably 1 ≤ n ≤ 4, and m in each case = 2 or 3.

4. Process according to Claim 1, **characterised in that** the difluorotris(perfluoroalkyl)phosphorane employed is a compound selected from the group consisting of difluorotris(pentafluoroethyl)phosphorane, difluorotris(n-nonafluorobutyl)phosphorane and difluorotris-(n-heptafluoropropyl)phosphorane.

5. Process according to Claim 1, **characterised in that** the trifluorobis-(perfluoroalkyl)phosphorane compound employed is trifluorobis-(n-nonafluorobutyl)phosphorane.

6. Process according to Claim 1, **characterised in that** the temperature during the heating in process step b) is room temperature to 150°C, preferably 100°C to 145°C, particularly preferably 135 to 140°C.

7. Process according to Claim 1, **characterised in that** the duration of the heating in process step b) is 1 to 150 hours, preferably 10 to 25 hours, particularly preferably 18 to 22 hours.

8. Process according to Claim 1, **characterised in that** the reaction medium is water or a water-based mixture.

9. Process according to Claim 2, **characterised in that** bases, preferably hydroxides, oxides, hydrides, amides, carbonates, phosphines or amines, are used to prepare the salts.

10. Salts of bis(perfluoroalkyl)phosphinic acids selected from the group of partially alkylated and peralkylated ammonium, phosphonium, sulfonium, pyridinium, pyridazinium, pyrimidinium, pyrazinium, imidazolium, pyrazolium, thiazolium, oxazolium and triazolium salts, apart from triethylmethylammonium bis(heptafluoropropyl)phosphinate and apart from N-methyl-2-trifluoromethylbenzothiazolium bis(heptafluoropropyl)phosphinate.

11. Salts of bis(perfluoroalkyl)phosphinic acids according to Claim 10, having a cation selected from the group 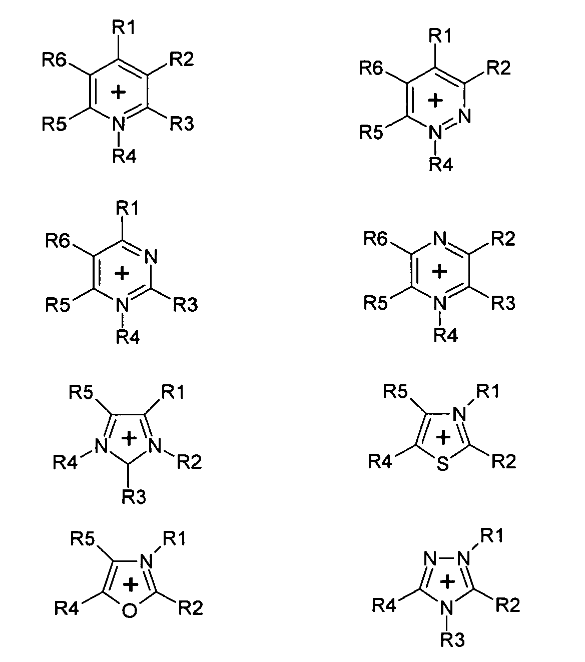 where R¹ to R⁶ are identical or different, are optionally bonded directly to one another by a single or double bond and each, individually or together, have the following meaning:
- H,
- halogen, where the halogens are not bonded directly to N,
- an alkyl radical (C₁ to C₈), which may be partially or completely substituted by further groups, preferably F, Cl, N(CₙF₍₂ₙ₊₁₋ₓ₎Hₓ)₂, O(CₙF₍₂ₙ₊₁₋ₓ)Hₓ), SO₂(CₙF(₂ₙ₊₁₋ₓ)Hₓ), CₙF₍₂ₙ₊₁₋ₓ₎Hₓ, where 1 < n < 6 and 0 < x ≤ 2n+1,
apart from N-methyl-2-trifluoromethylbenzthiazolium bis(heptafluoropropyl)phosphinate.

12. Use of the salts of bis(perfluoroalkyl)phosphinic acids according to Claim 10 or 11 as ionic liquids.

13. Use of the salts of bis(perfluoroalkyl)phosphinic acids according to Claim 10 or 11 as phase-transfer catalyst or surfactants.

## Revendications

1. Procédé de préparation d'acides bis(perfluoroalkyl)phosphiniques ou de sels de ceux-ci, comprenant au moins les étapes de procédé suivantes :
a) la réaction d'au moins un difluorotris(perfluoroalkyl)phosphorane ou d'au moins un trifluorobis(perfluoroalkyl)phosphorane avec du fluorure d'hydrogène dans un milieu réactionnel convenable, et
b) le chauffage du milieu réactionnel obtenu en a).

2. Procédé de préparation d'acides bis(perfluoroalkyl)phosphiniques ou de sels de ceux-ci selon la revendication 1, **caractérisé en ce que** les sels sont préparés par neutralisation ultérieure.

3. Procédé selon la revendication 1, **caractérisé en ce que** le difluorotris(perfluoroalkyl)phosphorane ou le trifluorobis(perfluoroalkyl)-phosphorane employé est un composé de formule générale I 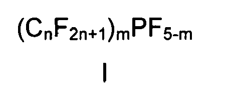 dans laquelle 1 ≤ n ≤ 8, préférablement 1 ≤ n ≤ 4, et m dans chaque cas = 2 ou 3.

4. Procédé selon la revendication 1, **caractérisé en ce que** le difluorotris(perfluoroalkyl)phosphorane employé est un composé choisi parmi le groupe constitué par le difluorotris(pentafluoroéthyl)-phosphorane, le difluorotris(n-nonafluorobutyl)phosphorane et le difluorotris(n-heptafluoropropyl)phosphorane.

5. Procédé selon la revendication 1, **caractérisé en ce que** le composé de trifluorobis(perfluoroalkyl)phosphorane employé est le trifluorobis(n-nonafluorobutyl)phosphorane.

6. Procédé selon la revendication 1, **caractérisé en ce que** la température pendant le chauffage dans l'étape de procédé b) va de la température ambiante à 150°C, préférablement de 100°C à 145°C, particulièrement préférablement de 135 à 140°C.

7. Procédé selon la revendication 1, **caractérisé en ce que** la durée du chauffage dans l'étape de procédé b) va de 1 à 150 heures, préférablement de 10 à 25 heures, particulièrement préférablement de 18 à 22 heures.

8. Procédé selon la revendication 1, **caractérisé en ce que** le milieu réactionnel est de l'eau ou un mélange à base d'eau.

9. Procédé selon la revendication 2, **caractérisé en ce que** des bases, préférablement des hydroxydes, des oxydes, des hydrures, des amides, des carbonates, des phosphines ou des amines, sont utilisées pour préparer les sels.

10. Sels d'acides bis(perfluoroalkyl)phosphiniques choisis parmi le groupe constitué par les sels d'ammonium, de phosphonium, de sulfonium, de pyridinium, de pyridazinium, de pyrimidinium, de pyrazinium, d'imidazolium, de pyrazolium, de thiazolium, d'oxazolium et de triazolium partiellement alkylés et peralkylés, à l'exception du bis(heptafluoropropyl)phosphinate de triéthylméthylammonium et à l'exception du bis(heptafluoropropyl)phosphinate de N-méthyl-2-trifluorométhylbenzothiazolium.

11. Sels d'acides bis(perfluoroalkyl)phosphiniques selon la revendication 10, ayant un cation choisi parmi le groupe constitué par 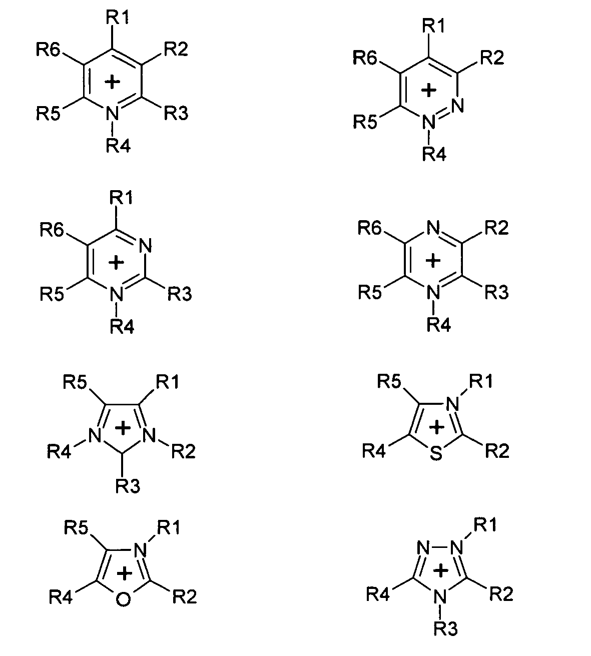 où R¹ à R⁶ sont identiques ou différents, sont éventuellement liés directement les uns aux autres par une liaison simple ou double et revêtent chacun, individuellement ou ensemble, la signification suivante :
- H,
- halogène, où les halogènes ne sont pas liés directement à N,
- un radical alkyle (C₁ à C₈), pouvant être partiellement ou complètement substitués par des groupements supplémentaires, préférablement F, CI, N(CₙF₍₂ₙ₊₁₋ₓ₎Hₓ)₂, O(CₙF(₂ₙ₊₁₋ₓ₎Hₓ), SO₂(CₙF₍₂ₙ₊₁₋ₓ₎Hₓ), CₙF(₂ₙ₊₁₋ₓ)Hₓ, où 1 < n < 6 et 0 < x ≤ 2n+1,
à l'exception du bis(heptafluoropropyl)phosphinate de N-méthyl-2-trifluorométhylbenzothiazolium.

12. Utilisation des sels d'acides bis(perfluoroalkyl)phosphiniques selon la revendication 10 ou 11, comme liquides ioniques.

13. Utilisation des sels d'acides bis(perfluoroalkyl)phosphiniques selon la revendication 10 ou 11, comme catalyseurs de transfert de phase ou agents tensioactifs.
